Europäisches Patentamt

European Patent Office

Office européen des brevets

(19)

(11) Publication number: 0 297 866
A2

(12) **EUROPEAN PATENT APPLICATION**

(21) Application number: 88305941.2

(22) Date of filing: 30.06.88

(51) Int. Cl.⁴: **A 61 K 9/20**
A 61 K 9/22, A 61 K 9/26

(30) Priority: 01.07.87 US 68845

(43) Date of publication of application:
04.01.89 Bulletin 89/01

(84) Designated Contracting States:
BE DE ES FR GB IT NL

(71) Applicant: THE BOOTS COMPANY PLC
1 Thane Road West
Nottingham NG2 3AA (GB)

(72) Inventor: Chen, Jivn-Ren
7614 Brook Haven Way
Shreveport, LA 71105 (US)

Defesche, Charles
509 Loch Ridge Drive
Shreveport, LA 71106 (US)

(74) Representative: Thacker, Michael Anthony et al
THE BOOTS COMPANY PLC Patents Section R4
Pennyfoot Street
Nottingham NG2 3AA (GB)

(54) Therapeutic agents.

(57) Sustained release granules of ibuprofen comprising a homogeneous mixture of ibuprofen and a pharmaceutically acceptable polymeric material, operable to provide a slow release of ibuprofen over a pH range from 1.0 to 7.0, the polymeric material being present in an amount of 5% to 25% by weight, based on the weight of the ibuprofen. The granules are prepared by dissolving the ibuprofen and polymeric material in a solvent and evaporating the solvent. Suitable polymeric materials include ethyl cellulose, high viscosity hydroxy propyl methyl cellulose and copolymers of dimethyl aminoethyl methylacrylate and methacrylic acid ester. The granules may be compressed to form sustained release tablets.

EP 0 297 866 A2

Bundesdruckerei Berlin

0 297 866

## Description

## THERAPEUTIC AGENTS

The present invention relates to sustained release therapeutic compositions comprising ibuprofen and a pharmaceutically acceptable polymeric material. The sustained release therapeutic compositions of the present invention are oral dosage formulations providing a long lasting and pre-determined incremental release of ibuprofen upon oral administration. Ibuprofen, the chemical name of which is 2-(4-isobutylphenyl)propionic acid, is a well known medicament with anti-inflammatory, analgesic and anti-pyretic properties.

Sustained release compositions are well known in the art and are described, for example in Christenson et al US patent 3065143, "Formulating Sustained Release Pharmaceutical Products with Methocel.", the Dow Chemical Company 1982 and Lapidus et al, J. Pharma. Sci., Vol 57, no.8, August 1968 1292-1301. Tansey US patent 3133863, discloses the preparation of a timed release formulation of phenobarbital in which a solution of ethyl cellulose in methylene chloride and ethyl alcohol is used to granulate a uniform blend of phenobarbital, methyl cellulose and lactose, the resulting granules being compressed into tablets. The amount of ethyl cellulose is 41.7% by weight, based on the weight of phenobarbital. Schor et al, US patent 4389393 is concerned with the preparation of sustained release formulations based on particular high molecular weight hydroxypropyl methyl celluloses such as Methocel K4M and Methocel K15M (Methocel is a trade name of the Dow Chemical Company). A specific example discloses controlled release ibuprofen tablets containing 700 mg ibuprofen, Methocel K4M and Methocel K15M and other excipients, the tablets being prepared by dry blending of the ingredients and direct compression of the resulting mixture. The combined amount of Methocel K4M and Methocel K15M is 23.5% by weight, based on the weight of ibuprofen.

Polymers have also been used in the preparation of pharmaceutical compositions for uses other than sustained release. For example, Showa Shinyaku Co, Japanese patent application no.87/126122 discloses a method for the surface coating of ibuprofen granules in which water is added to a stirred solution of ibuprofen and an acrylic acid type resin in the presence of a surface active agent to form granules of ibuprofen surface coated with the resin. CERTA, Belgian patent 839625 discloses a method for the enteric coating of a medicament such as erythromycin by crystallisation of the coated medicament from a solution of the medicament and a coating agent such as cellulose acetate phthalate or a copolymer of methacrylic acid and methacrylic acid ester.

However, the prior art techniques available for the production of sustained release therapeutic compositions are not entirely satisfactory to furnish a sustained release tablet capable of delivering 600-800 mg of active ingredient as is required for a sustained release dosage of ibuprofen. Thus for the administration of ibuprofen by means of a sustained release composition on a twice daily dosage, with preferably no more than 1 to 2 tablets per dose, the dosage form should preferably contain no less than 600 mg of ibuprofen. The present invention is based upon the provision, in an advantageous manner, of sustained release compositions capable of delivering these large daily dosages.

In accordance with the present invention, there is provided a sustained release pharmaceutical composition which comprises granules comprising a homogeneous mixture of ibuprofen and a pharmaceutically acceptable polymeric material operable to provide a slow release of ibuprofen over a pH range of 1.0 to 7.5, the polymeric material being present in an amount of 5% to 25% by weight, based on the weight of ibuprofen.

The present invention also provides a process for the preparation of sustained release granules comprising ibuprofen and a pharmaceutically acceptable polymeric material operable to provide a slow release of the ibuprofen over the pH range of 1.0 to 7.5, the polymeric material being present in an amount of 5% to 25% by weight, based on the weight of the ibuprofen, which comprises dissolving the ibuprofen and the polymeric material in a solvent and evaporating the solvent to form granules of co-precipitated ibuprofen and polymeric material.

The process of the present invention may be carried out by dissolving the pharmaceutically acceptable polymeric material and the ibuprofen in a solvent and removing the solvent to form a semi-dry mass as by evaporating the solvent under mild conditions under vacuum. The semi-dry mass is granulated to form small, uniform granules which can be made into tablets by direct compression. Alternatively, the solvent may be evaporated by spray-drying the solution. In a further method the polymeric material can be dissolved in a solvent and the ibuprofen added to the solution to form a paste which can be granulated to obtain small, uniform particles. The granules of the present invention can be formed into tablets by direct compression. Regardless of the method used to form the coprecipitated granules, the granules thus obtained comprise a homogeneous mixture of the polymeric material and the ibuprofen. Thus the granules are homogeneous both in an outer layer and an inner portion.

The granules thus obtained can be formulated into sustained release dosage forms, such as tablets, coated tablets, capsules and the like with or without the incorporation of additives conventionally used in such compositions, such as magnesium stearate, lactose, and in general, binders, fillers, disintegrating agents, channelling agents and the like.

The process for forming the coprecipitated granules used in the present invention provides a large amount of small, individual polymer/ibuprofen complex granules. Each individual granule itself is, in essence, a miniature drug delivery system in which the on the surface of the granule will be quickly released whereas the ibuprofen in the interior of the granule will be more slowly released. Accordingly, the small granules

2

themselves may be employed in oral dosage compositions to advantage. For example, it is well known that accumulation of ibuprofen in the gastrointestinal tract or even the passage of an intact solid dosage unit of ibuprofen through the gastrointestinal tract may cause irritation thereof. The use of an oral dosage form comprising the small coprecipitated granules themselves, such as are released from disintegratable tablets, will provide a large number of granules spread out over a large area of the mucosa of the gastrointestinal tract. The use of the large amount of the small granules will not only minimise the presence of high local concentrations of ibuprofen on the irritable mucosa, but it will also avoid the risks of drug accumulation or the passage of an intact solid dosage unit of large size through the gastrintestinal tract.

When the small granules obtained through the present invention are formed into a sustained release composition, the ibuprofen on the surface of the composition will be quickly released whereas the ibuprofen in the core of the tablet or other sustained release composition will be more slowly and more gradually released. The polymeric material will be uniformly distributed throughout the sustained release composition to form a matrix in which the ibuprofen is embedded.

The sustained release compositions of the present invention offer numerous advantages over the sustained release compositions of the prior art. As described above, the process of producing the coprecipitated granules is a simple and direct process that is cost efficient. The granules obtained through the invention may be directly compressed into sustained release tablets using conventional tableting equipment and technology. In addition, the coprecipitated granules according to the invention offer the dual function of fast release of ibuprofen from the surface of the granules and/or tablets containing the granules and slow release of ibuprofen embedded in the interior of the granules and/or tablets. Through the choice of proportions of polymeric material and ibuprofen, as well as the choice of polymeric material itself and the various additives employed in sustained release compositions, the ibuprofen release profile of the sustained release compositions of the present invention can be controlled and "tailored".

Polymeric materials which are operable to produce a slow release of ibuprofen over the pH range from 1.0 to 7.5 and hence can be used in accordance with the present invention include:

| Chemical Name | Trade Name |
|---|---|
| High viscosity hydroxy propyl-methyl cellulose | Methocel K4M |
| | Methocel K15M |
| Ethyl cellulose | Ethocel |
| Dimethylaminoethyl methacrylate and methacrylic acid ester copolymers | Eudragit RL |
| | Eudragit RS |

Methocel and Ethocel are trade names of the Dow Chemical Company. Eudragit is a trade name of Rohm Pharma.

The polymeric materials listed above are pharmaceutically acceptable and they are operable to provide a slow release over a pH range encountered in the stomach and intestine, namely over the pH range of 1.0 to 7.5.

A wide variety of organic solvents may be employed in the formation of the coprecipitated granules comprising ibuprofen and the polymeric material, such as methylene chloride, chloroform, ethanol, isopropanol, acetone and mixtures of two or more solvents, and the like. Usually an amount of solvent will be used sufficient to dissolve the materials added thereto and the use of larger amounts than needed is not economically desirable.

The amount of polymeric material used is preferably from 5% to 15% by weight based on the weight of ibuprofen.

Generally, the granules and/or sustained release compositions containing the granules will also contain one or more of the additives mentioned above, such as the materials listed below:

3

| Chemical Name | Trade Name |
|---|---|
| Low-substituted hydroxypropyl cellulose | L-HPC |
| Sodium carboxymethyl cellulose or its cross-linked form | Ac-Di-Sol |
| Calcium carboxymethyl cellulose starch | Sta-Rx 1500 |
| Sodium starch glycolate | Primojel |
| Polyvinyl pyrrolidone (PVP) | Plasdone |
| or polyvinyl pyrrolidone | Polyplastdone XL |
| Microcrystalline cellulose | Avicel |
| Lactose | |
| Sucrose | |
| Fibrous cellulosic derivative | CLD |
| Micron-sized amorphous silica gel | Syloid |

The additives can be added to the solution of the polymeric material and the medicament, or they can be dry-blended with the granules prior to tableting. The amount of the additive will be determined empirically from case-to-case, but generally the total amount of additives will be from about 5% to about 20%, preferably from about 6% to about 15%, based upon the total weight of the composition.

The preparation of the coprecipitated granules is direct and straightforward. The desired amounts of ibuprofen and the polymeric material are dissolved in the solvent, and, if desired, the materials can be stirred and/or heated to a moderate temperature, for example from about 30 to about 40°C, to assist in the dissolution of all of the ingredients. If necessary, insoluble particles can be removed by filtration, such as through the use of a fritted glass disc filter or the like. The solution is then subject to removal of the solvent, as by vacuum evaporation or spray-drying. Moderate temperatures of from about 30 to about 60°C may be used during the vacuum evaporation to accelerate the formation of the coprecipitated granules. The process thus described results in the formation of a semi-dried mass, which can be granulated by passage through a screen of desired mesh size. Alternatively, the granules can be formed by dissolving the polymer in a suitable organic solvent and then adding the therapeutically active medicament to the solution to form a paste, which is dried and granulated as described above.

The present invention is illustrated by the following Examples. In this specification and the accompanying claims, all parts and proportions are by weight, unless otherwise stated.

Example 1

Coprecipitated granules containing ibuprofen were prepared by dissolving 800 gm ibuprofen and 80 gm Eudragit RL grade in 1.5 litres of acetone under gentle stirring. Acetone was removed by heating the solution to about 40-55°C under vacuum (about 700 mm Hg). The semi-dried mass thus obtained was passed through a 14 mesh screen to obtain granules containing 800 mg ibuprofen. The completely dry granules were then passed through a 18 mesh screen to obtain uniform size granules.

Examples 2-10

Following the procedure of Example 1, coprecipitated granules of ibuprofen and polymer were prepared and these granules were dry blended with magnesium stearate and other additives and directly compressed into tablets containing 800 mg ibuprofen and having a hardness of 10-17 S.C. depending on the tablet strength. The ingredients used to form the granules and the tablets are set forth below for each Example.

Example 2

|  | Grams |
|---|---|
| Granules | 92.0 |

    Ibuprofen        - 80 gm  
    Methocel K4M    - 6.0 gm  
    Methocel K15M   - 6.0 gm

| | |
|---|---|
| Lactose, hydrous | 4.0 |
| Microcrystalline cellulose | 2.0 |
| Magnesium stearate | 0.5 |

Example 3a

| | |
|---|---|
| Granules | 92.0 |

    Ibuprofen      - 80 gm  
    Methocel K4M   - 12 gm

| | |
|---|---|
| Lactose, fast flow | 6.0 |
| Magnesium stearate | 0.5 |

Example 3b

| | | |
|---|---|---|
| Granules | | 92.0 |
|     Ibuprofen | - 80 gm | |
|     Methocel K15M | - 12 gm | |
| | | |
| Lactose, spray dried | | 6.0 |
| Magnesium stearate | | 0.5 |

Example 4a

| | | |
|---|---|---|
| Granules | | 88.0 |
|     Ibuprofen | - 80 gm | |
|     Methocel K4M | -  8 gm | |
| | | |
| Lactose, hydrous | | 10.0 |
| Magnesium stearate | | 0.5 |

Example 4b

| | | |
|---|---|---|
| Granules | | 88.0 |
|     Ibuprofen | - 80 gm | |
|     Methocel K15M | -  8 gm | |
| | | |
| Lactose, hydrous | | 10.0 |
| Magnesium stearate | | 0.5 |

Example 5

| Granules | 88.0 |
|---|---|

    Ibuprofen      - 80 gm
    Eudragit RL   - 4.0 gm
    Eudragit RS   - 4.0 gm

| Lactose, hydrous | 6.0 |
|---|---|
| Microcrystalline cellulose | 4.0 |
| Magnesium stearate | 0.5 |

Example 6

| Granules | 88.0 |
|---|---|

    Ibuprofen      - 80 gm
    Eudragit RL   - 8 gm

| Lactose, hydrous | 10.0 |
|---|---|
| Magnesium stearate | 0.5 |

Example 7

| Granules | 88.0 |
|---|---|

    Ibuprofen      - 80 gm
    Eudragit RL   - 8 gm

| Microcrystalline cellulose | 8.0 |
|---|---|
| Low-substituted hydroxypropyl cellulose | 2.0 |
| Magnesium stearate | 0.5 |

Example 8

| | | | |
|---|---|---|---|
| Granules | | | 92.0 |
|     Ibuprofen | - | 80 gm | |
|     Eudragit RL | - | 6 gm | |
|     Eudragit RS | - | 6 gm | |

| | |
|---|---|
| Microcrystalline cellulose | 5.0 |
| Low-substituted hydroxypropyl | |
|   cellulose | 1.0 |
| Magnesium stearate | 0.5 |

Example 9

| | | | |
|---|---|---|---|
| Granules | | | 92.0 |
|     Ibuprofen | - | 80 gm | |
|     Methocel K4M | - | 4 gm | |
|     Methocel K15M | - | 8 gm | |

| | |
|---|---|
| Lactose, fast flow | 6.0 |
| Magnesium stearate | 0.5 |

Example 10

| | | | |
|---|---|---|---|
| Granules | | | 88.0 |
|     Ibuprofen | - | 80 gm | |
|     Ethylcellulose | - | 8 gm | |

| | |
|---|---|
| Lactose, spray dried | 5.0 |
| Sodium carboxymethylcellulose | 3.0 |
| Starch 1500 | 2.0 |
| Magnesium stearate | 0.5 |

The tablets of Examples 7, 8 and 10 contained disintegrating agents and hence were disintegrating tablets, whereas the tablets of the other Examples are "matrix tablets" that slowly release the medicament from their polymeric matrix in which the medicament is embedded.

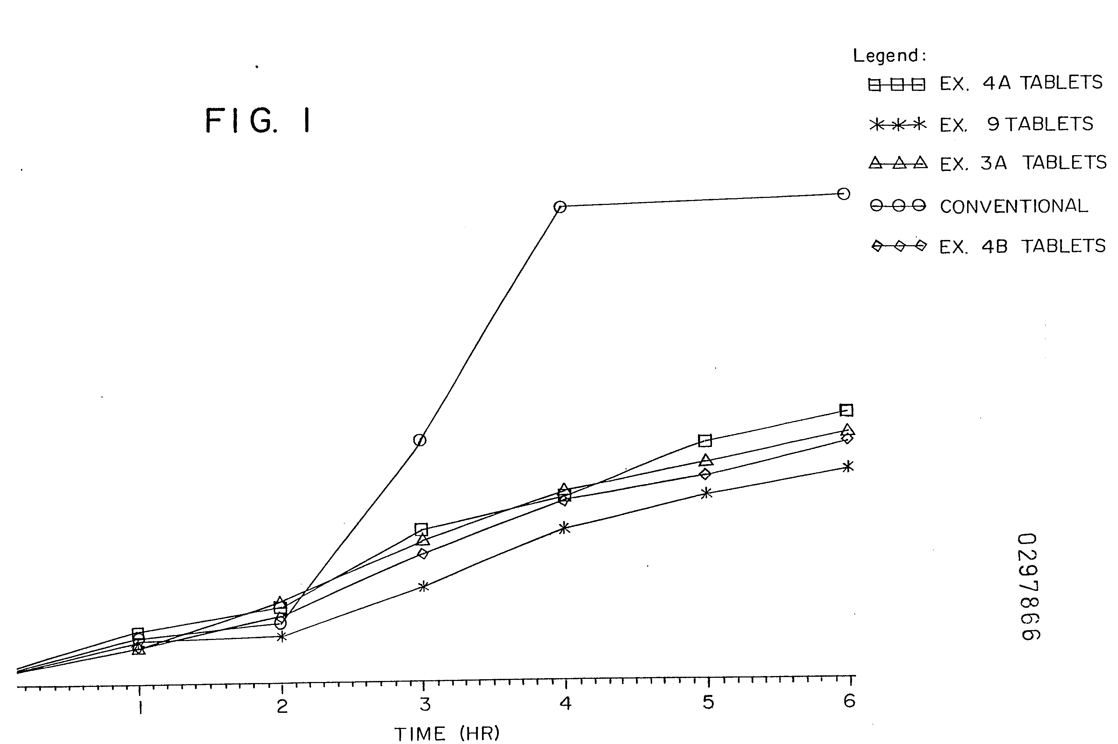

The rate of dissolution of the matrix tablets of Examples 3a, 4a, 4b, and 9 was determined and is reported in Figure 1 of the accompanying drawings. For convenience, the identity and proportion of the polymeric material used to form the granules for these matrix tablets are set forth below:

## Parts by Weight

| Example | Ibuprofen | Methocel K4M | Methocel K15M |
|---------|-----------|--------------|---------------|
| 4a | 100 | 10 | -- |
| 3a | 100 | 15 | -- |
| 4b | 100 | -- | 10 |
| 9 | 100 | 5 | 10 |

The tablets of the above Examples were placed in 900 ml of a pH 2.0 buffer solution[1] and a 10 ml sample of the liquid was removed and analysed for ibuprofen. After taking the 1st hour sample, a suitable amount of ($37° \pm 0.2°C$) alkaline buffer solution[1] was added to adjust the dissolution medium to a new pH value. A suitable amount of $37° \pm 0.2°C$ distilled water was also added to substitute the sample value of 10 ml if necessary. Samples were taken hourly and analysed by a spectrophotometer at 265 mm. The amount of ibuprofen released was calculated as a percentage of the total content of the tablet (or granules). The pH adjustments of the dissolution medium are made according to the following schedule.

| Time Hour | pH of Dissolution Medium |
|-----------|--------------------------|
| 1st | $2.0 \pm 0.2$ |
| 2nd | $4.5 \pm 0.2$ |
| 3rd | $6.0 \pm 0.2$ |
| 4th | $7.5 \pm 0.2$ |
| 5th | $7.5 \pm 0.2$ |
| 6th | $7.5 \pm 0.2$ |

The sum of the amount of distilled water used for replacement and the amount of alkaline buffer solution used equalled 10 ml.

The data of Figure 1 demonstrate that the rate of release of medicament from the granules can be predetermined depending upon the nature and amount of the polymer. In particular, Figure 1 shows the effect of the polymer viscosity and concentration on the dissolution rate profile of the ibuprofen-Methocel matrix tablets. These results indicate the granule forms a matrix in tablets providing a slow- release system for the drug. The varying viscosity of the polymers and the slight increase of the polymer concentration do not significantly alter the drug release profile in this matrix system.

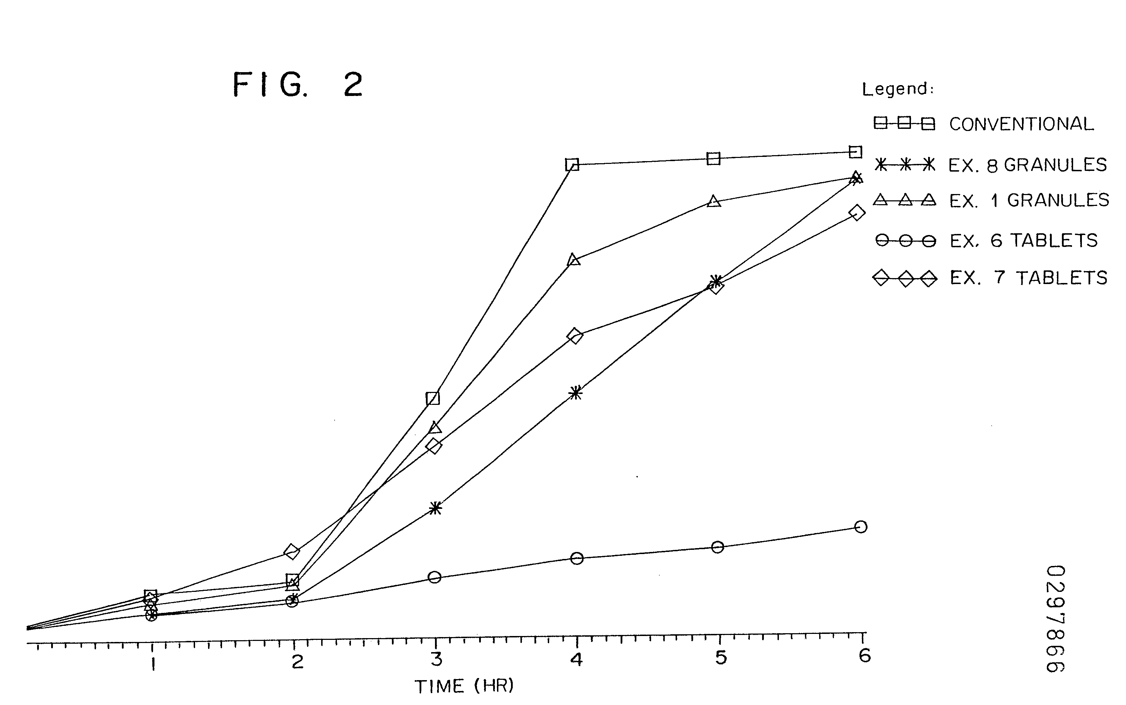

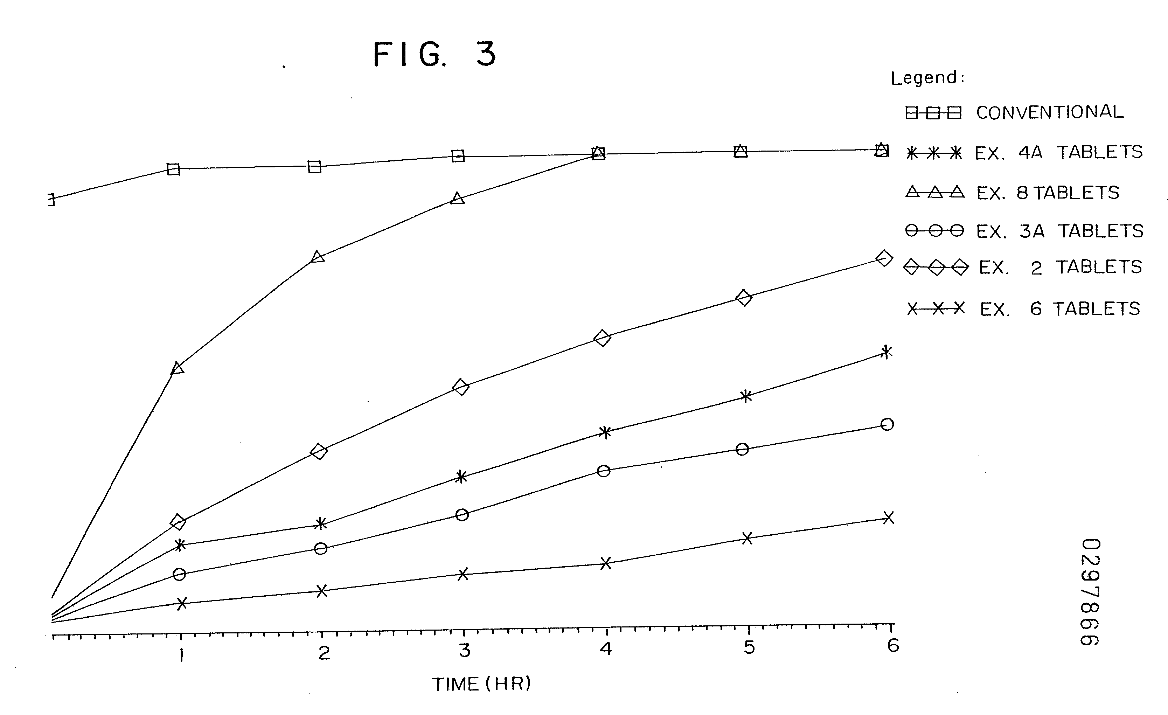

To establish a drug release profile for the Eudragit/drug composition, the granules of Example 1, the granules of Example 8, the tablets of Example 6 and the tablets of Example 7 were evaluated. Figure 2 illustrates the comparative dissolution profile of ibuprofen-Eudragit granules in various systems. The drug in the granules shows a slower release rate than conventional tablets. Evidently the granules sustain the drug release beyond a certain extent. The distinct difference between the profile of the matrix tablet (Example 6) and disintegrating tablet (Example 7) provides a wide range for managing the desirable release rate. This

[1] pH 2.0 buffer solution-Dissolve 3 grams $KH_2PO_4$ in 900 ml of distilled water and adjust the pH of this solution to 2 with concentrated HC1 acid. Alkaline buffer solution - dissolved 100 gm of NaOH in 1 litre of distilled water. Mix well.

noticeable advantage arises from the coprecipitated granules of the present invention.

The rate of dissolution of the tablets of Examples 8, 2, 4a, 3a and 6 was determined in comparison with that of conventional tablets. A modified dissolution method with 50 RPM of Apparatus II for ibuprofen tablets (USP XXI, page 527) was used. A 10 ml sample was pipetted out of the dissolution medium at hourly time periods (except for conventional release tablets) and replaced by the same quantity of $37°C \pm 0.2°C$ pH 7.2 buffer solution. The filtrate of the sample was assayed by a spectrophotometer at 265 nm and the released amount of ibuprofen was calculated as a percentage of the total content.

These results show that the release rate of drug from tablets made with co-crystallisation granules of the invention can range from slow (Example 6) to fast (Example 8) in a predetermined manner. By adjusting the combination of the different type, viscosity and concentration of polymers in the granules of the present invention, a desirable controlled release dosage form can be prepared.

## Claims

1. A sustained release pharmaceutical composition which comprises granules comprising a homogeneous mixture of ibuprofen and a pharmaceutically acceptable polymeric material operable to provide a slow release of ibuprofen over a pH range from 1.0 to 7.5, the polymeric material being present in an amount of 5% to 25% by weight, based on the weight of the ibuprofen.

2. A pharmaceutical composition according to claim 1 in which the polymeric material is present in an amount of 5% to 15% by weight, based on the weight of the ibuprofen.

3. A pharmaceutical composition according to claim 1 or claim 2 in which the polymeric material comprises ethyl cellulose, a copolymer of dimethylaminoethyl methylacrylate and methacrylic acid ester or high viscosity hydroxy propyl methyl cellulose.

4. A pharmaceutical composition according to claim 3 in which the polymeric material comprises ethyl cellulose.

5. A pharmaceutical composition according to any one of claims 1 to 4 in the form of a tablet comprising said granules.

6. A tablet which comprises granules comprising a homogeneous mixture of ibuprofen and ethyl cellulose, the ethyl cellulose being present in an amount of 5% to 15% by weight, based on the weight of ibuprofen.

7. A tablet according to claim 6 in which the ethyl cellulose is present in an amount of 10% by weight, based on the weight of ibuprofen.

8. A tablet according to claim 6 or claim 7 also comprising a disintegrating agent.

9. A process for the preparation of sustained release granules comprising ibuprofen and a pharmaceutically acceptable polymeric material operable to provide a slow release of the ibuprofen over the pH range 1.0 to 7.5, the polymeric material being present in an amount of 5% to 25% by weight, based on the weight of ibuprofen, which comprises dissolving the ibuprofen and the polymeric material in a solvent and evaporating the solvent to form granules of coprecipitated ibuprofen and polymeric material.

10. A process according to claim 9 for the production of granules according to any one of claims 1 to 4.

11. A process for the production of tablets which comprises the compression of granules prepared by the process of claim 10.

12. A process according to claim 11 for the production of tablets according to claim 6.

**Claims for the following Contracting State : ES**

1. A process for the preparation of sustained release granules comprising ibuprofen and a pharmaceutically acceptable polymeric material operable to provide a slow release of the ibuprofen over the pH range 1.0 to 7.5, the polymeric material being present in an amount of 5% to 25% by weight, based on the ibuprofen, which comprises dissolving the ibuprofen and the polymeric material in a solvent and evaporating the solvent to form granules of coprecipitated ibuprofen and polymeric material.

2. A process according to claim 1 in which the polymeric material is present in an amount of 5% to 15% by weight, based on the weight of the ibuprofen.

3. A process according to claims 1 or 2, in which the polymeric material comprises ethyl cellulose, a copolymer of dimethylaminoethyl methylacrylate and methacrylic acid ester or high viscosity hydroxy propyl methyl cellulose.

4. A process according to claim 3 in which the polymeric material comprises ethyl cellulose.

5. A process for the production of tablets comprising compression of granules prepared according to claim 1.

6. A process for the production of tablets which comprises the compression of granules prepared according to claim 2.

7. A process for the production of tablets comprising the compression of granules prepared according to claim 3.

8. A process for the production of tablets which comprises the compression of granules prepared according to claims 1 to 3 together with a disintegrating agent.

FIG. 1

Legend:
⊟⊟⊟ EX. 4A TABLETS
✳✳✳ EX. 9 TABLETS
△△△ EX. 3A TABLETS
⊖⊖⊖ CONVENTIONAL
◇◇◇ EX. 4B TABLETS

TIME (HR)

0297866

# FIG. 2

Legend:

☐–☐–☐  CONVENTIONAL

✳–✳–✳  EX. 8 GRANULES

△–△–△  EX. 1 GRANULES

⊖–⊖–⊖  EX. 6 TABLETS

◇–◇–◇  EX. 7 TABLETS

TIME (HR)

# FIG. 3

Legend:
⊟⊟⊟ CONVENTIONAL
✳✳✳ EX. 4A TABLETS
△△△ EX. 8 TABLETS
⊖⊖⊖ EX. 3A TABLETS
◇◇◇ EX. 2 TABLETS
✕✕✕ EX. 6 TABLETS

TIME (HR)

0297866